# EUROPÄISCHE PATENTSCHRIFT

(11) **EP 0 634 175 B1**
(45) Veröffentlichungstag und Bekanntmachung des Hinweises auf die Patenterteilung: **10.01.2001**
(21) Anmeldenummer: 94110064.6
(22) Anmeldetag: 29.06.1994
(51) Int. Cl.: A61K 38/55, A61K 45/06

(54) **Pharmazeutische Kombination, die einen Hemmer des Renin-Angiotensin-Systems und einen Endothelin-Antagonisten enthält**
Pharmaceutical combination, containing a renin-angiotensin-system inhibitor and an endothelin antagonist
Combinaison pharmaceutique contenant un inhibiteur du système rénin-angiotensin et un antagoniste d'endothéline

(30) Priorität: 15.07.1993 CH 213193
(43) Veröffentlichungstag der Anmeldung: 18.01.1995
(73) Patentinhaber: F. HOFFMANN-LA ROCHE AG, 4002 Basel (CH)
(72) Erfinder: Clozel, Jean-Paul, F-68300 St. Louis (FR); Clozel, Martine, F-68300 St. Louis (FR); Osterrieder, Wolfgang, deceased (DE)
(74) Vertreter: Witte, Hubert, Dr.

(56) Entgegenhaltungen:
- J. HYPERTENS. (UNITED KINGDOM), VOL. 10, SUPPL. 7 (S121-S132,, 1992, VOL. 10, SUPPL. 7 (S121-S132, Hedner T. et al 'Peptides as targets for antihypertensive drug development'

## Beschreibung

Die vorliegende Erfindung betrifft pharmazeutische Kombinationspräparate, die zur Behandlung von Herz-Kreislauf-Erkrankungen geeignet sind und einen Hemmer des Renin-Angiotensin-Systems (RAS-Hemmer) und einen Endothelin-Antagonisten enthalten.

Das Renin-Angiotensin-System spielt eine bedeutende Rolle bei der Regulierung des Blutdrucks. Es lässt sich als eine proteolytische Kaskade beschreiben, die sich in folgende Schritte aufteilt:
1. Das Enzym Renin spaltet Angiotensinogen in Angiotensin I, das biologisch inaktiv ist.
2. Angiotensin I wird durch das Enzym ACE (Angiotensin Converting Enzyme) in Angiotensin II umgewandelt.
3. Angiotensin II bindet an Rezeptoren in der Membran von Effektorzellen und löst eine biologische Antwort aus.

Diese biologischen Antworten sind vielfältig, beispielsweise Kontraktion von Blutgefässen, Aldosteronbiosynthese und Stimulierung von Gefässwachstum nach einer Schädigung.

Das Renin-Angiotensin-System lässt sich in allen drei oben beschriebenen Schritten hemmen, nämlich im ersten durch die Anwendung eines Reninhemmers, im zweiten durch die Anwendung eines ACE-Hemmers und im dritten durch die Anwendung eines Antagonisten des Angiotensin II-Rezeptors.

In einem Uebersichtsartikel im J. of Hypertension, vol. 10, suppl.7, 121-131 (1992) werden Verbindungen beschrieben, welche das Renin-Angiotensin-System in den spezifischen Schritten hemmen.

In der Behandlung des Bluthochdrucks und der Herzinsuffizienz haben ACE-Hemmer eine grosse Bedeutung erlangt. Der Prototyp für diese Substanzklasse ist Captopril. Reninhemmer und Antagonisten des Angiotensin II-Rezeptors sind bisher noch nicht auf dem Markt, doch wird an deren Entwicklung weltweit gearbeitet.

Endothelin ist ein erst vor wenigen Jahren entdecktes Peptidhormon [Yanagisawa et al., Nature 332, 411 - 415 (1988)], welches sich als bisher potentester Vasokonstriktor erwiesen hat. Endothelin wird von den die Blutgefässe auskleidenden Endothelzellen sekretiert. Obwohl dessen Konzentration im Blutplasma sehr niedrig ist, kann davon ausgegangen werden, dass lokale Konzentrationen zwischen Endothelzellen und den benachbarten glatten Muskelzellen der Gefässe wesentlich höher liegen.

Erhöhte Endothelinspiegel im Blutplasma findet man bei einer Reihe von Herz-Kreislauf-Erkrankungen, wie Hypertonie, Herzinsuffizienz, Ischämie (Herz, Gehirn, Magen-Darm-Trakt und Niere) oder Vasospasmen. Bei Patienten mit Asthma ist die Konzentration von Endothelin im Bronchialsekret erhöht. Auch bei Migräneanfällen findet man erhöhte Endothelinspiegel im Blutplasma.

Endothelin bindet an spezifische Rezeptoren der Effektorzellen. Bisher sind zumindest zwei Untertypen dieser Rezeptoren, nämlich Endothelin _{A}- und Endothelin _{B}-Rezeptoren beschrieben worden [Lin et al., Proc. Natl. Acad. Sci. USA 88, 3185 - 3189 (1991) und Sakamoto et al., Biochem. Biophys. Res. Commun. 178, 656 - 663 (1991)]. Endothelin _{A} bewirkt die Vasokonstriktion, während die Bedeutung von Endothelin _{B} noch nicht ausreichend geklärt ist.

Wegen der pathophysiologischen Bedeutung von Endothelin besteht das Bedürfnis, einen geeigneten Endothelin-Antagonisten bereitzustellen. In beispielsweise den Europäischen Patentpublikationen 405.421, 436.189, 457.195, 460.679 und 496.452 sowie J. Antibiotics 45, 74 (1992), FEBS 305, 41 (1992) und Bioch. Biophys. Res. Comm. 185, 630 (1992) sind eine Reihe von Verbindungen beschrieben, welche die Bindung von Endothelin an den Rezeptor hemmen. Es handelt sich hier allerdings entweder um Substanzen, die in Gärbrühen gefunden wurden und chemisch praktisch nicht herstellbar sind, oder um Peptide, die sich mit grösster Wahrscheinlichkeit wegen ungenügender Bioverfügbarkeit nicht für eine orale Anwendung am Menschen eignen. Hingegen sind die in den Europäischen Patentpublikationen 510.526 und 526.708 sowie WO 93/08 799 beschriebenen Endothelin-Antagonisten von einfacher chemischer Struktur und besitzen den Vorteil der oralen Anwendbarkeit.

Im Rahmen der vorliegenden Erfindung konnte festgestellt werden, dass bei Verabreichung der erfindungsgemässen Kombination eines Hemmers des Renin-Angiotensin-Systems mit einem Endothelin-Antagonisten die blutdrucksenkenden Eigenschaften und die Wirkungsdauer der Einzelkomponenten nicht nur addiert, sondern überraschenderweise potenziert werden, wodurch die wirksamen Dosen der beiden Einzelkomponenten signifikant herabgesetzt werden können.

Die erfindungsgemässe Kombination besitzt demnach die Vorteile, dass die zu applizierenden Wirkstoffmengen signifikant reduziert und unerwünschte Nebenwirkungen eliminiert bzw. stark reduziert werden können.

Die erfindungsgemässe Kombination kann demnach als Mittel zur Behandlung von Erkrankungen verwendet werden, die mit einer Vasokonstriktion oder anderen biologischen Wirkungen von Endothelin und/oder Angiotensin II assoziiert sind. Beispiele solcher Erkrankungen sind Bluthochdruck, Koronarerkrankungen, Herzinsuffizienz, renale und myokardiale Ischämie, Niereninsuffizienz, Dialyse, subarachnoidale Hämorrhagie, Raynaud-Syndrom und pulmonarer Hochdruck. Dazu gehört auch die Behandlung von Magen- und Duodenalulcera sowie von Ulcus cruris, bei denen eine Vasokonstriktion beteiligt ist.

Sie können ebenfalls bei Atherosklerose und zur Verhinderung der Restenosierung nach Ballon-induzierter Gefässdilatation eingesetzt werden.

Gegenstand der vorliegenden Erfindung sind somit
- eine Kombination von einem RAS-Hemmer und einem Endothelin-Antagonisten
- eine pharmazeutische Zubereitung, enthaltend einen RAS-Hemmer und einen Endothelin-Antagonisten
- die Herstellung einer pharmazeutischen Zubereitung, welche dadurch gekennzeichnet ist, dass man ein Gemisch von einem RAS-Hemmer und einem Endothelin-Antagonisten in eine galenische Darreichungsform bringt
- die Verwendung einer Kombination von einem RAS-Hemmer und einem Endothelin-Antagonisten bzw. einer pharmazeutischen Zubereitung, enthaltend einen RAS-Hemmer und einen Endothelin-Antagonisten zur gleichzeitigen, getrennten oder zeitlich abgestuften Anwendung bei der Behandlung von Erkrankungen, die mit einer Vasokonstriktion oder anderen biologischen Wirkungen von Endothelin und/oder Angiotensin II assoziiert sind, insbesondere von Kreislauferkrankungen, ganz besonders bei der Bekämpfung bzw. Verhütung von Hypertension und deren Folgeerkrankungen sowie bei der Behandlung von Herzinsuffizienz.
- Eine Handelspackung die eine Kombination von einem RAS-Hemmer und einem Endothelin-Antagonisten bzw. eine pharmazeutische Zubereitung enthaltend einen RAS-Hemmer und einen Endothelin-Antagonisten beinhaltet, mit Instruktionen für die Verwendung dieser Kombination der Wirkstoffe.

Das Gewichtsverhältnis vom RAS-Hemmer zum Endothelin-Antagonisten beträgt zweckmässigerweise 1:1 bis 1:500, vorzugsweise 1:1 bis 1:100.

Vorteilhafterweise beträgt die mittels einer Kombination pro Tag zu verabreichende Dosis 2,5 bis 10 mg eines RAS-Hemmers und 250 bis 1000 mg eines Endothelin-Antagonisten. Im allgemeinen beträgt die täglich zu verabreichende Gesamtmenge von einem RAS-Hemmer und einem Endothelin-Antagonisten maximal 550 mg. Wird ein Hydrat oder ein pharmazeutisch verwendbares Salz eingesetzt, so sind die obigen Werte entsprechend zu ändern.

Als Hemmer des Renin-Angiotensin-Systems kommen Reninhemmer, ACE-Hemmer und Angiotensin II-Antagonisten in Frage. Bevorzugt ist die Verwendung von ACE-Hemmern.

Beispiele von ACE-Hemmer sind Alacepril, Benazepril, Captopril, Cilazapril, Cilazaprilat, Delapril, Enalapril, Enalaprilat, Fosinopril, Lisinopril, Perindopril, Quinapril, Ramipril, Spirapril, Zofenopril und MC 838 [Calciumsalz von (R-(R,S)-1-(3-((2-((Cyclohexylcarbonyl)amino)-1-oxopropyl)thio)-2-methyl-1-oxopropyl)-L-prolin] sowie Analoga dieser Verbindungen wie sie in den Europäischen Patentpublikationen EPA 7.477, 12.401, 50.800, 51.391, 53.902, 65.301, 72.352, 94.095, 172.552, 211.220 und 271.795, den US Patentschriften 4.105.776 und 4.316.906, der Britischen Patentschrift 2.102.412 sowie Tetrahedron Letters, 23, 1677 - 1680 (1982) beschrieben sind. Der geeignete ACE-Hemmer ist das Cilazapril.

Beispiele von Reninhemmer sind:

### Ciprokiren

(S)-2-Benzyl-N-[(S)-1-[(1S,2R,3S)-1-cyclohexylmethyl-3-cyclopropyl-2,3-dihydroxypropylcarbamoyl]-2-(imidazol-4-yl)ethyl]-3-[1-methyl-- 1-(morpholin-4-ylcarbonyl)ethylsulfonylmethyl]propionamid;

### Ditekiren

Boc-Pro-Phe-N-His-Leu-(CHOH-CH₂)-Val-Ile-Asp;

### Enalkiren

[1S-(1R*,2S*,3R*)]-N-(3-Amino-3-methyl-l-oxobutyl)-O-methyl-L-tyrosyl-N-[1-(cyclohexylmethyl)-2,3-dihydroxy-5-methylhexyl]-L-histidinamid;

### Remikiren

(S)-2-tert-Butylsulfonylmethyl-N-[(S)-1-[(1S,2R,3S)-1-cyclohexylmethyl-3-cyclopropyl-2,3-dihydroxypropylcarbamoyl]-2-(1H-imidazol-4-yl)methyl]-3-phenylprionamid;

### Terlakiren

[R-(R*,S*)]-N-(4-Morpholinylcarbonyl)-L-phenylalanyl-N-[1-(cyclohexylmethyl)-2-hydroxy-3-(1-methylethoxy)-3-oxopropyl]-S-methyl-L-cysteinamid;

### A 65 317

N-[2(R)-Benzyl-N-[2-[2-(2-methoxyethoxy)methoxy]ethyl]-N-methylsuccinamoyl]histidin 1(S)-cyclohexylmethyl-2(R)-hydroxy-2-[3-ethyl-2-oxo-oxazolidin-5(S)-yl]ethylamid;

### A 72 517

N-[2(S)-Benzyl-3-(4-methyl-1-piperazinylsulfonyl)propanoyl]-3-(4-thiazolyl)-L-alanine 1(S)-(cyclohexylmethyl)-2(R),3(S)-dihydroxy-5-methylhexylamid;

### A 70 461

N-[2(S)-Amino-1-cyclohexyl-3(R),4(S)-dihydroxy-6-methylhept-1-yl]-2(S)-[1(S)-[4-(methoxymethoxy)piperidin-1-ylcarbonyl]-2-phenylethoxy]hexanamid;

### A 74 273

6-Cyclohexyl-4(S)-hydroxy-2(S)-isopropyl-5(S)-[2(S)-[1(S)-[4-(methoxy-methoxy)piperidin-1-ylcarbonyl]-2-phenylethoxy]hexanamido]-N-(3-morpholinopropyl)hexanamid;

### A 82 110

6-Cyclohexyl-4(S)-hydroxy-2(S)-isopropyl-5(S)-[N-[1(S)-[4-(methoxy-methoxy)piperidin-1-ylcarbonyl]-2-phenylethyl]-L-norleucylamino]-N-(2-methyl-2-morpholinopropyl)hexanamid;

### WAY 121 604

(3S)-4-Cyclohexyl-2-hydroxy-3-[[(2S)-4-methyl-2-[[(2S)-2-(1-oxo-1,3-dihydroisoindol-2-yl)-3-phenylpropionyl]amino]pentanoyl]amino]buttersäuremethylester;

### SQ 31 844

[R-(R*,S*)]-N-(4-Morpholinylcarbonyl)-L-phenylalanyl]-N-[1-(cyclohexylmethyl)-2-hydroxy-2-(1H-imidazol-2-yl)ethyl]-L-histidinamid;

### SQ 33 800

[1S-[1R*(R*),2S*,3S*]]-[2-(tert-Butylsulfonylmethyl)-1-oxo-3-phenylpropyl]-N-[4-[(butylamino)sulfonyl]-1-(cyclohexylmethyl)-2,3-dihydroxybutyl]-L-histidinamid-monomethanesulfonat;

### GR 70 982

[1S-(1R*,2R*,4S*)]-N-[(1,1-Dimethylethoxy)carbonyl]-L-phenylalanyl-N-[5-[(4-aminobutyl)amino]-1-(cyclohexylmethyl)-2-hydroxy-5-oxo-4-(4-pyridinylmethyl)pentyl]-L-histidinamid;

### ICI 219 623

(2S,4S,5S)-N-Butyl-6-cyclohexyl-4-hydroxy-2-isopropyl-5-[2-[8-propyl-6-(3-pyridyl)-1,2,4-triazolo[4,3-a]pyrazin-3-yl]-3-(3-pyridyl)propionamido]-hexanamid;

### L 157 119

4-(Boc-Phe-His-ACHPA-Lys-NH-CH₂)pyridin;

### CP 71 362

[2R-(2R*,4S*,5S*)]-N-[N2-[6-Cyclohexyl-5-[[N-[N-[(1,1-dimethylethoxy)carbonyl]-L-phenylalanyl]-L-histidyl]amino]-4-hydroxy-2-(2-methylpropyl)-1-oxohexyl]-L-lysyl]- L-phenylalanin-diacetat;

### ES 8 891

5-Cyclohexyl-2,4,5-trideoxy-N-hexyl-4-[[N-[3-(1-naphthalenyl)-N-(4-morpholinylacetyl)-L-alanyl]-3-(4-thiazolyl)-L-alanyl]amino]-L-threopentanamid;

### ES 1 005

[1S-[1R*(R*),2R*,4[R*(R*)]]]-N-[4-[[1-[[(5-Amino-6-hydroxyhexyl)amino]carbonyl]-3-methylbutyl]amino]-2-hydroxy-1-(2-methylpropyl)-4-oxobutyl]-α-[[3-(1-naphthalenyl)-2-(1-naphthalenylmethyl)-1-oxopropyl]amino]-1H-imidazol-4-propanamid;

### PD 132 002

[1S-(1R*,2S*,3R*)]-N-(4-Morpholinylsulfonyl)-L-phenylalanyl-N-[1-(cyclohexylmethyl)-2,3-dihydroxy-5-methylhexyl]-O-methyl-3-oxo-D- oder L-serinamid;

### PD 134 674

([1S-(1R*,2S*,3R*)])-N-(4-Morpholinylsulphonyl)-L-phenylalanyl-3-(2-amino-4-thiazolyl)-N-[(cyclohexylmethyl)-2,3-dihydroxy-5-methylhexyl]-L-alaninamid;

### YM 21 095

N-[2-[[1-(Cyclohexylmethyl)-2-hydroxy-3-[(1-methyl-1H-tetrazol-5-yl)thio]propyl]amino]-l-(1H-imidazol-4-ylmethyl)-2-oxoethyl]-α-(1-naphthalenylmethyl)-γ-oxo-4-morpholinobutanamid;

### FK 906

N-Methyl-N-[2(S)-[N-methyl-N-[2-[N-methyl-N-(morpholinocarbonyl)amino]ethyl]carbamoyl]-3-phenylpropionyl]-L-histidin 1(S)-(cyclohexylmethyl)-2(S)-hydroxy-5-methylhexylamid;

### FK 744

[1S-[1R*[R*(R*)],2R*]]-Methyl [3-(4-morpholinyl)-3-oxopropyl] 2-[[2-[[1-(cyclohexylmethyl)-2-hydroxy-5-methylhexyl]amino]-1-(1H-imidazol-4-ylmethyl)-2-oxoethyl]methylamino]-2-oxo-1-(phenylmethyl)carbaminsäureethylester,

### S 89-2864

3-(4-Aminopiperidin-1-ylcarbonyl)-2(R)-benzylpropionyl-L-histidin 1(S)-(cyclohexylmethyl)-2(R),3(S)-dihydroxy-5-(2-pyridyl)pentylamid-acetat;

### MDL 73 323

3-Amino-3-methylbutyryl-(4-O-methyl)-L-tyrosyl-L-norvaline 1-(cyclohexylmethyl)-3,3-difluoro-4-(3-methylbutyramido)-2-oxobutylamid-hydrochloridhydrat; und

### JTP 3 071

1H-Indol-2-ylcarbonyl-L-histidin 1(S)-(cyclohexylmethyl)-2(S),4(S)- dihydroxy-5-methylhexylamid.

Beispiele von Angiotensin II-Antagonisten sind:

### Losartan

Kaliumsalz von 2-Butyl-4-chlor-1-[[2'-(1H-tetrazol-5-yl)[1,1'-biphenyl]-4-yl]methyl]-1H-imidazol-5-methanol;

### Valsartan

N-(1-Oxopentyl)-N-[[2'-( 1H-tetrazol-5-yl)[1,1'-biphenyl]-4-yl]methyl]-L-valin;

### BIBS 39

4'-[(2-n-Butyl-6-cyclohexylaminocarbonylamino-benzimidazol-1-yl)methyl]-biphenyl-2-carbonsäure;

### BIBS 222

2-n-Butyl-1-[4-(6-carboxy-2,5-dichlorbenzoylamino)benzyl]-6-N-(methylaminocarbonyl)-n-pentylaminobenzimidazol;

### BIBR 277

4'-[(1,4'-Dimethyl-2'-propyl-[2,6'-bi-1H-benzimidazol]-1'-yl)methyl]-[1,1'-biphenyl]-2-carbonsäure;

### DuP 532

4-(Pentafluorethyl)-2-propyl-1-[[2'-(1H-tetrazol-5-yl)[1,1'-biphenyl]-4-yl]methyl]-1H-imidazol-5-carbonsäure;

### EXP 7 711

4'-[[2-Butyl-4-chlor-5-(hydroxymethyl)-1H-imidazol-1-yl]methyl]-[1,1'-biphenyl]-2-carbonsäure;

### D 6 888

2-Ethyl-5,6,7,8-tetrahydro-4-([2'-(1H-tetrazol-5-yl)biphenyl-4-yl]methoxy)chinolin;

### D 8 731

2-Ethyl-4-[(2'-(1H-tetrazol-5-yl)biphenyl-4-yl)methoxy]chinolin-hydrochlorid;

### GR 117 289

1-((3-Brom-2-(2-(1H-tetrazol-5-yl)phenyl)-5-benzofuranyl)methyl)-2-butyl-4-chlor-1H-imidazol-5-carbonsäure;

### GR 138 950

1-[[3-Brom-2-[2-[[(trifluormethyl)sulphonyl]amino]phenyl]-5-benzofuranyl]methyl]-4-cyclopropyl-2-ethyl-1H-imidazol-5-carboxamid;

### L 158 809

5,7-Dimethyl-2-ethyl-3-(2'-(1H-tetrazol-5-yl)(1,1'-biphenyl)-4-yl)methyl)-3H-imidazo-(4,5-b)pyridin;

### SC 50 560

5-[4'-(3,5-Dibutyl-1,2,4-triazol-1-ylmethyl)biphenyl-2-yl]-1H-tetrazol;

### SC 51 895

1,4-Dibutyl-3-[2'-(1H-tetrazol-5-yl)biphenyl-4-ylmethyl]-2,3-dihydro-1H-imidazol-2-on;

### SR 47 436

2-n-Butyl-4-spirocyclopentan-1-[(2'-(1H-tetrazol-5-yl)biphenyl-4-yl)methyl]-2-imidazolin-5-on;

### SKF 108 566

(E)-α-[[2-Butyl-1-[(4-carboxyphenyl)methyl]-1H-imidazol-5-yl]methylen]-2-thiophenpropansäure;

### TCV 116/CV 11 974

2-Ethoxy-1-[[2'-(1H-tetrazol-5-yl)[1,1'-biphenyl]-4-yl]methyl]-1H-benzimidazol-7-carbonsäure-1-[[(cyclohexyloxy)carbonyl]oxy]ethylester;

### CI 996

2-Propyl-4-[(3-trifluoracetyl)pyrrol-1-yl]-1-[[2'-(2H-tetrazol-5-yl)[1,1'-biphenyl]-4-yl]methyl]-1H-imidazol-5-carbonsäure; und

### YM 358

Kaliumsalz von 2,7-Diethyl-5-[[2'(1H-tetrazol-5-yl)[1,1'-biphenyl]-4-yl]methyl]-5H-pyrazolo[1,5-b][1,2,4]triazol.

Als Endothelin-Antagonisten kommen peptidische und nicht-peptidische Derivate in Frage, wobei wegen der weiter oben erwähnten schlechten Bioverfügbarkeit, die bei den peptidischen Derivaten erwartet werden muss, die nicht-peptidischen Derivate bevorzugt sind. Besonders bevorzugt sind diejenigen nicht-peptidischen Derivate, die orale Wirksamkeit aufweisen. Beispiele von peptidischen Derivaten sind:

### FR 139 317;

Perhydroazepin-1-ylcarbonyl-L-leucyl-(1-methyl)-D-tryptophyl-[3-(2-pyridyl)]-D-alanin;

### FR 901367;

2-Acetamido-3-[[1,4,4a,5,6,6a,7,12,12a,12b-decahydro-4a,8,12a,12b-tetrahydroxy-3-methyl-1,7,12-trioxobenz[a]anthracen-6a-yl]thio]propionsäure;

### BE 18 257 B;

Cyclo(-D-Trp-D-Glu-L-Ala-allo-D-Ile-L-Leu-);

### BQ 123

Cyclo(-D-Trp-D-Asp-L-Pro-D-Val-L-Leu-);
Perhydroazepin-1-ylcarbonyl-L-leucyl-D-tryptophyl-D-tryptophan;
Cochinmicin I;
Myriceron-kaffeesäureester; und

### PD 142 893

Acetyl-(3,3-diphenyl-D-alanin)-L-Leu-L-Asp-L-Ile-L-Ile-L-Trp.

Beispiele von nicht-peptidische Endothelin-Antagonisten sind z.B. die Sulfonamide und die Indan- und Indenderivate die in den Europäischen Patentpublikationen 510.526 und 526.708 bzw. WO 93/08 799 beschrieben sind, insbesondere die Verbindungen:
4-tert-Butyl-N-[6-(2-hydroxyethoxy)-5-(2-methoxyphenoxy)-2,2'-bipyrimidin-4-yl]benzolsulfonamid;
(1RS, 2SR, 3SR)-1-(4-Methoxyphenyl)-3-(3,4-methylendioxyphenyl)-indan-2-carbonsäure;
(1RS, 2RS, 3SR)-5-Hydroxy-3-(4-methoxyphenyl)-1-(3,4-methylendioxyphenyl)indan-2-carbonsäure;
(1RS, 2RS, 3SR)-5-Methoxy-3-(4-methoxyphenyl)-1-(3,4-methylendioxyphenyl)indan-2-carbonsäure;
(1RS, 2SR, 3SR)-1,3-Bis(3,4-methylendioxyphenyl)-5-hydroxyindan-2-carbonsäure;
(1RS,2SR,3RS)-3-(2-Carboxymethoxy-4-methoxyphenyl)-1-(3,4-methylendioxyphenyl)-5-(prop-1-yloxy)indan-2-carbonsäure;
(1RS,2SR,3SR)-3-(2-Carboxymethoxy-4-methoxyphenyl)-1-(2-methoxy-4,5-methylendioxyphenyl)-5-(prop-1-yloxy)indan-2-carbonsäure;
(1RS,2SR, 3RS)-3-[2-(1-Carboxyeth-2-yloxy)-4-methoxyphenyl]-1-(3,4-methylendioxyphenyl)-5-(prop-1-yloxy)indan-2-carbonsäure-bis-dicyclohexylaminsalz;
(1RS, 2SR, 3SR)-[2-[(E)-2-Carboxyethen-1-yl]-4-methoxyphenyl]-1-(3,4-methylendioxyphenyl)-5-(prop-1-yloxy)indan-2-carbonsäure;
(1RS, 2SR, 3SR)-3-[2-(2-Carboxyeth-1-yl)-4-methoxyphenyl]-1-(3,4-methylendioxyphenyl)-5-(prop-1-yloxy)indan-2-carbonsäure und
(1RS, 2SR, 3RS)-3-[2-(3-Carboxyphenyl)-4-methoxyphenyl]-1-(3,4-methylendioxyphenyl)-5-(prop-1-yloxy)indan-2-carbonsäure.

Erfindungsgemäss von besonderem Interesse sind Kombinationen von 4-tert-Butyl-N-[6-(2-hydroxyethoxy)-5-(2-methoxyphenoxy)-2,2'-bipyrimidin-4-yl]benzolsulfonamid (nachstehend auch Verbindung A genannt) und Cilazapril.

Mit der erfindungsgemässen Kombination kann mit geringen Wirkstoffdosen eine regelmässige und lang andauernde blutdrucksenkende Wirkung erzielt werden.

Die vorteilhafte überadditive blutdrucksenkende Wirkung sowie die verlängerte Wirkungsdauer der erfindungsgemässen Kombination gegenüber derjenigen der beiden Einzelkomponenten soll anhand der nachfolgend wiedergegebenen Versuche gezeigt werden.

### Blutdrucksenkung in spontan hypertonen Ratten

Eine erste Studie wurde mit Ratten durchgeführt. Verwendet wurden Ratten des Stammes SHRSP (Gewicht ca. 300 g und 18 - 20 Wochen alt). SHRSP (sponanteously hypertensive rats, stroke prone) ist die gängige Bezeichnung für spontan hypertone Ratten, die mit dem Alter eine Neigung zum Gehirnschlag entwickeln. Im Alter von 18 Wochen hat sich bei diesen Ratten der Blutdruck stabilisiert.

An diesen Ratten wurde der arterielle Blutdruck mit einer telemetrischen Methode gemessen. Das telemetrische System wird von Data Sciences, Inc., St. Paul, Minn., USA vertrieben. Die Blutdruckmessung erfolgte mittels eines flüssigkeitsgefüllten Katheters, der in die abdominale Aorta implantiert worden war. Die Implantierung erfolgte unter einer Kurznarkose (15 - 20 min) mit Evipan® (100 mg/kg intraperitoneal). Der Katheter war mit einem Minisender in der Bauchhöhle verbunden, der die Blutdruckschwankungen zu einem Sender ausserhalb des Käfigs sendete. Auf diese Weise war eine kontinuierliche Messung in frei beweglichen Tieren über einen längeren Zeitraum möglich.

Die Studie wurde mit einer Gesamtzahl von 9 Ratten in einem Cross-Over-Design durchgeführt, d.h. jede dieser Ratten erhielt einmal keine Substanz (nur Lösungsmittel), einmal Verbindung A, einmal Cilazapril und einmal die Kombination von Verbindung A mit Cilazapril. Zwischen den einzelnen Verabreichungen lag eine Zeit von mindestens 48 Stunden. Die Dosierung war 100 mg/kg oral für Verbindung A und 1 mg/kg oral für Cilazapril. Die Verabreichung erfolgte mit einer Schlundsonde.

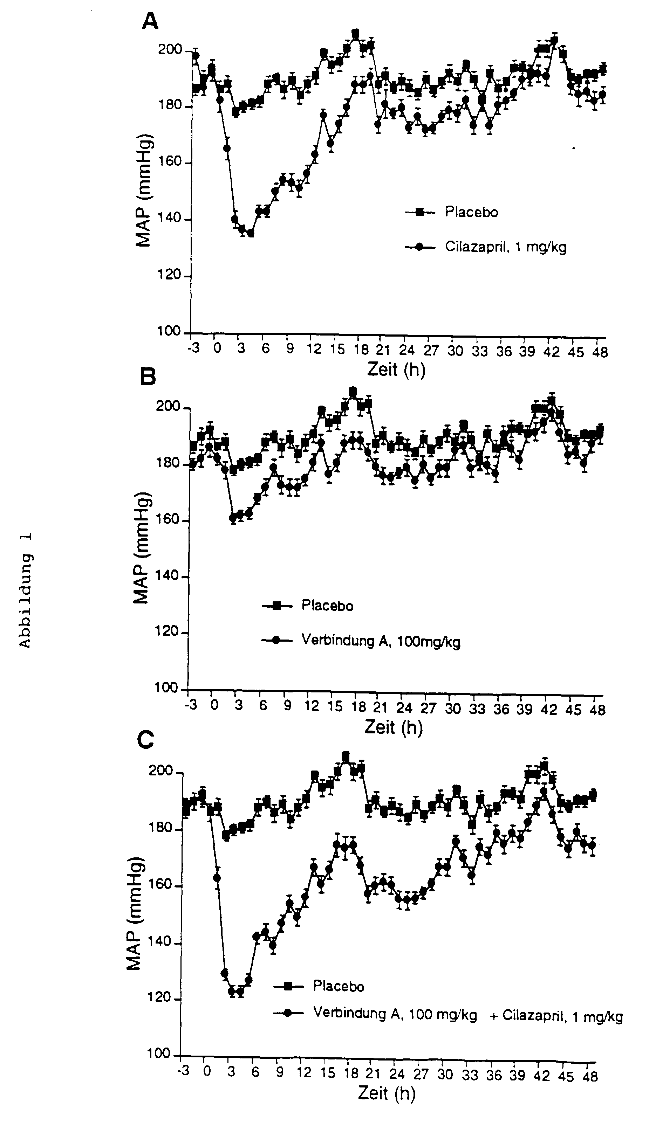

Die Abbildung 1 fasst die Ergebnisse dieser ersten Serie von Experimenten zusammen und illustriert die Wirkung von Cilazapril (1 mg/kg p.o.) und Verbindung A (100 mg/kg p.o.) allein, sowie die Wirkung der gleichzeitigen Verabreichung derselben Dosen der beiden Substanzen (n = 9 Ratten).

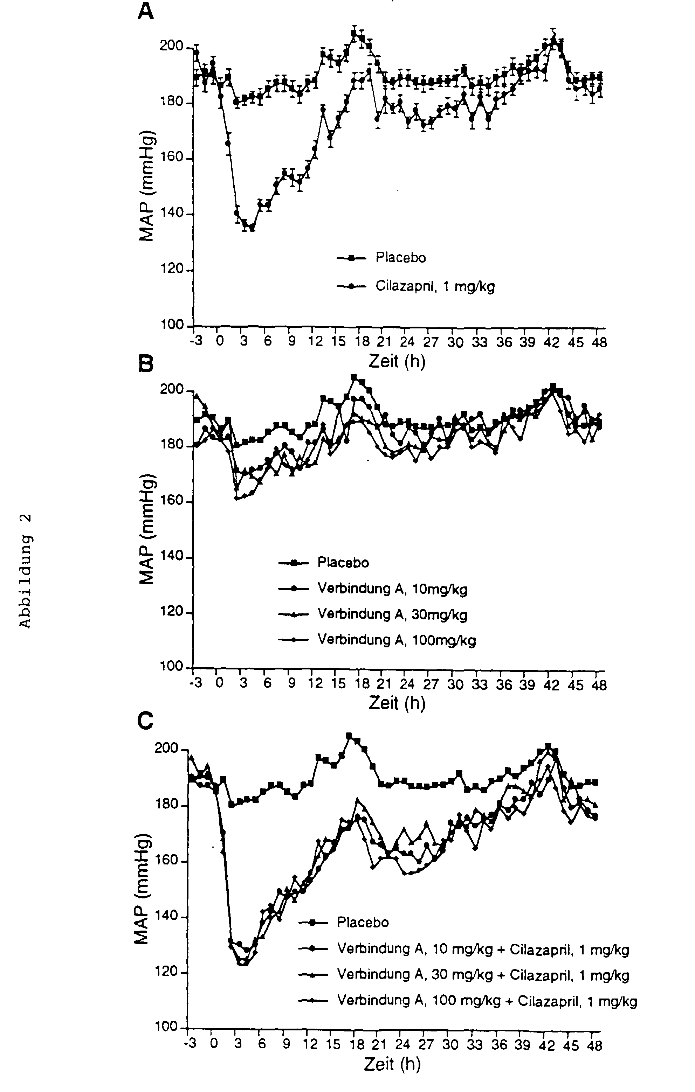

Zur Absicherung des erhaltenen Ergebnisses wurden weitere Versuche mit zwei anderen Dosierungen von Verbindung A durchgeführt, nämlich mit 10 und 30 mg/kg p.o., wobei die Dosis von Cilazapril von der ersten Versuchsreihe (Abbildung 1 C; 1 mg/kg p.o.) beibehalten wurde. Die erhaltenen Ergebnisse sind in der Abbildung 2 zusammengefasst, wobei die Daten mit Cilazapril allein sowie mit 100 mg/kg Verbindung A in die Abbildung 2 übernommen wurden.

Die Werte in den Abbildungen 1 und 2 sind die Mittelwerte der Blutdrucke in den 9 Versuchstieren, die Fehlerbalken geben den Standardfehler an. Aus Gründen der Klarheit sind die Fehlerbalken in den Abbildungen 2 B und 2 C weggelassen worden.

Verbindung A senkte den mittleren arteriellen Blutdruck (MAP) leicht, wobei der maximale Effekt etwa 20 mmHg betrug (Abb. 2B). Cilazapril zeigte die erwartete Drucksenkung, die ca. 20 Stunden anhielt (Maximaleffekt: Blutdruckreduktion um etwa 45 mmHg (Abb. 2A)). Die Kombination senkte den Blutdruck um etwa maximal 60 mmHg, wobei hervorzuheben ist, dass nach 20 Stunden immer noch ein deutlicher Effekt und noch 40 Stunden immer noch der gleiche Effekt wie mit Cilazapril allein nach 20 Stunden beobachtet werden konnte (Abb. 2C).

Die beiden tieferen Dosierungen von Verbindung A wirkten ähnlich wie die hohe (Abb. 2B). Die Wirkungsverlängerung könnte bestätigt werden.

### Blutdrucksenkung in normotonen Affen

In einer zweiten Studie wurde die Wirkung von Verbindung A in Kombination mit dem Reninhemmer Remikiren untersucht. Für diese Studie wurden Affen verwendet, da Remikiren spezifisch für Primatenrenin ist und an Ratten daher nicht wirkt. Remikiren ist ein potenter Reninhemmer mit oraler Verfügbarkeit (Fischli et al., Hypertension 18, 22-31 (1991)).

Die Messgrösse war der arterielle Blutdruck, der ebenfalls telemetrisch gemessen wurde. Die Affen der Spezies Saimiri sciureus (Totenkopfäffchen oder squirrel monkeys) wogen 400-700 g. Durch eine Natriumdepletion erhöht sich die Reaktivität des Blutdrucks auf RAS-Hemmer. Die Natriumdepletion wurde durch subkutane Injektion von 5 mg/kg Furosemid (Lasix®) 66, 42 und 18 Stunden vor Versuchsbeginn erreicht. In der Nacht vor dem Experiment bekamen die Tiere kein Futter. Während des Versuchs wurden die Affen in einem separaten Raum gehalten und mit Videokameras überwacht, um durch die Experimentatoren ausgelösten Stress mit resultierenden Blutdruckschwankungen zu vermeiden.

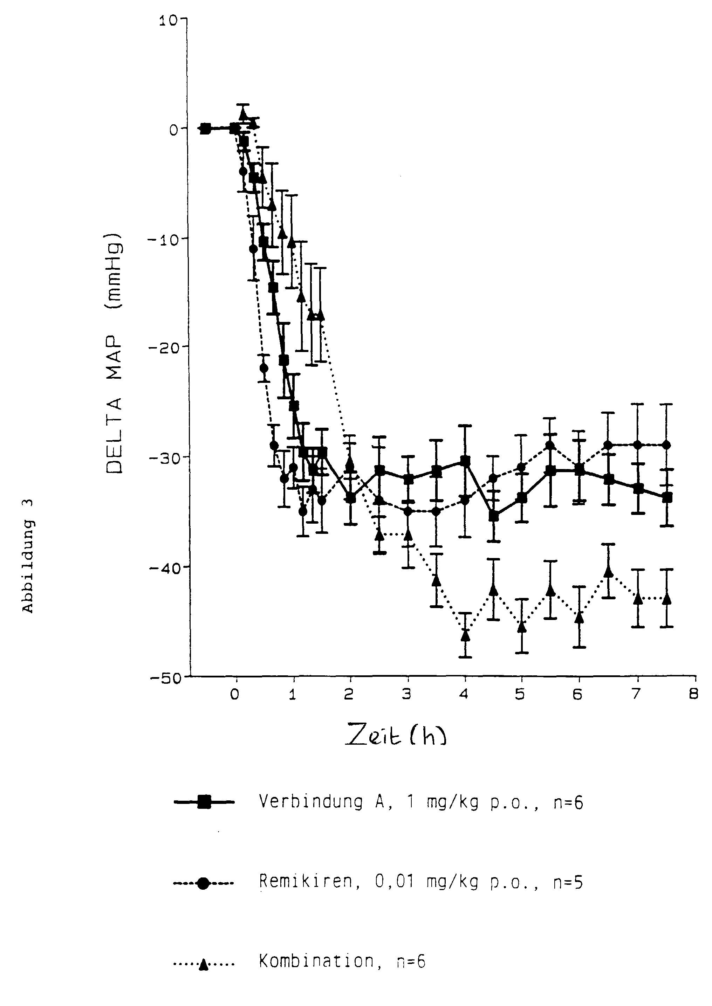

Die Abbildung 3 fasst die Ergebnisse dieser Versuche zusammen, wobei jeweils die Aenderungen des mittleren Blutdrucks, der bei 100 mmHg lag, angegeben sind. Verbindung A in einer Dosierung von 1 mg/kg p.o. (n = 6 Affen) und 0,01 mg/kg Remikiren (n = 5 Affen) senkten den Blutdruck um je etwa 30 mmHg. Die Kombination war deutlich effektiver. Da aus tierschützerischen Gründen die Affen nicht länger als 8 Stunden im Messkäfig gehalten werden dürfen, ist keine Aussage zur Wirkungsdauer möglich.

### Herzinsuffizienz

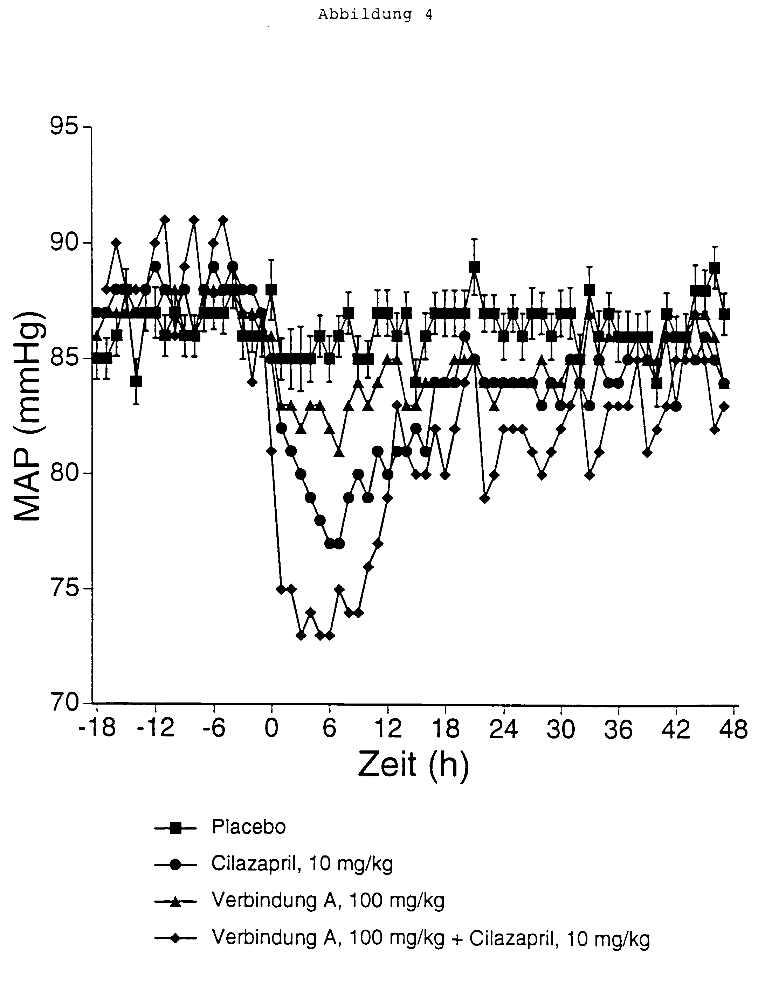

Da ACE-Hemmer heute in der Standardtherapie von Patienten mit Herzinsuffizienz eingesetzt werden, wurden die Verbindung A und Cilazapril einzeln und in Kombination auch noch in einem Tiermodell für Herzinsuffizienz geprüft. Verwendet wurden Ratten, bei denen sich 8 Wochen nach einer Koronarligatur (A. circumflex) die Symptome einer vollen Herzinsuffizienz eingestellt hatten. Der arterielle Blutdruck wurde, wie oben beschrieben, telemetrisch gemessen. Die Ergebnisse dieser Versuche sind in der Abbildung 4 zusammengefasst. Aus Gründen einer übersichtlicheren Darstellung wurden die Fehlerbalken nur bei der Placebokurve eingezeichnet. Auch in diesem Tiermodell bestätigte sich die Wirkungsverstärkung und -verlängerung der Kombination gegenüber den Einzelkomponenten.

Die vorstehenden Ergebnisse zeigen die unerwartet vorteilhaften Eigenschaften der erfindungsgemässen Kombinationen.

Die erfindungsgemässen Kombinationen werden im allgemeinen oral, z.B. in Form von Tabletten, Lacktabletten, Dragées, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen. Die Verabreichung der Wirkstoff kann in Form von Präparaten erfolgen, die beide Wirkstoffe in einer Dosiseinheitsform, wie Tabletten oder Kapseln enthalten, oder getrennt als ad-hoc Kombination von Dosiseinheitsformen, gleichzeitig oder zeitlich abgestuft.

Zur Herstellung von Tabletten, Lacktabletten, Dragées und Hartgelatinekapseln kann eine erfindungsgemässe Kombination mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Zubereitungen können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutische wertvolle Stoffe enthalten.

Die nachfolgenden Beispiele erläutern die Erfindung.

### Beispiel 1

Herstellung von Lacktabletten folgender Zusammensetzung:

| Lacktablettenkern: | |
|---|---|
| a. Verbindung A | 500.0 mg |
| b. Cilazapril | 5.0 mg |
| c. Lactose wasserfrei | 30.0 mg |
| d. Mikrokristalline Cellulose | 30.0 mg |
| e. Polyvinylpyrrolidon | 20.0 mg |
| f. Magnesiumstearat | 5.0 mg |
| Gewicht pro Lacktablettenkern | 590.0 mg |

| Lacküberzug: | |
|---|---|
| g. Hydroxypropylmethylcellulose | 3.5 mg |
| h. Aethylcellulose | 3.5 mg |
| i. Polyäthylenglykol 6000 | 0.8 mg |
| j. Eisenoxid gelb | 1.2 mg |
| k. Titandioxid | 0.3 mg |
| l. Talk | 0.7 mg |
| Gewicht Lacküberzug | 10.0 mg |
| Totalgewicht pro Lacktablette | 600.0 mg |

### Herstellungsverfahren:

### Herstellung des Lacktablettenkerns:

Verbindung A, Cilazapril, die Lactose, die Cellulose und das Polyvinylpyrrolidon werden gemischt und gesiebt. Die Mischung wird feuchtgranuliert, getrocknet und gesiebt. Das gesiebte Granulat wird mit dem Magnesiumstearat gemischt, und die pressfertige Mischung zu ovalen Tablettenkernen à 590.0 mg verpresst.

### Herstellung des Lacküberzugs:

Aus g bis l wird eine wässrige Lacksuspension hergestellt und damit die Lacktablettenkerne in geeigneter Weise mit Hilfe eines Lackierverfahrens in einem Dragierkessel oder einer anderen Lackierapparatur überzogen, bis die Lacktabletten ein Endgewicht von 600 mg erreicht haben.

### Beispiel 2

Herstellung von Hartgelatinekapseln folgender Zusammensetzung:

| | |
|---|---|
| a. Verbindung A | 250.0 mg |
| b. Cilazapril | 2.5 mg |
| c. Lactose krist. | 18.0 mg |
| d. Polyvinylpyrrolidon | 15.0 mg |
| e. Microkristalline Cellulose | 17.5 mg |
| f. Natrium-carboxymethylstärke | 10.0 mg |
| g.Talk | 9.0 mg |
| g. Magnesiumstearat | 3.0 mg |
| Füllgewicht pro Kapsel | 325.0 mg |

### Herstellungsverfahren:

Verbindung A, Cilazapril, die Lactose, das Polyvinylpyrrolidon und die Cellulose werden gesiebt und gemischt. Die Mischung wird feuchtgranuliert und getrocknet. Das Granulat wird mit der Natrium-carboxymethylstärke, dem Talk und dem Magnesiumstearat gemischt und die abfüllfertige Endmischung in Hartgelatinekapseln der Grösse 1 abgefüllt.

## Patentansprüche

1. Präparat, dadurch gekennzeichnet, dass es 4-tert-Butyl-N-[6-(2-hydroxyethoxy)-5-(2-methoxyphenoxy)-2,2'-bipyrimidin-4-yl]benzolsulfonamid und Cilazapril enthält.

2. Präparat gemäss Anspruch 1 zur Anwendung bei der Bekämpfung bzw. Verhütung von Hypertension und deren Folgeerkrankungen sowie bei der Behandlung von Herzinsuffizienz.

3. Präparat nach einem der Ansprüche 1 - 2, dadurch gekennzeichnet, dass das Gewichtsverhältnis vom RAS-Hemmer aus der Gruppe von ACE-Hemmer, zum nicht-pepti-dischen Endothelin-Antagonisten 1:1 bis 1:500 beträgt.

4. Präparat nach einem der Ansprüche 1 - 3, dadurch gekennzeichnet, dass es 2,5 bis 10 mg eines RAS-Hemmers aus der Gruppe von ACE-Hemmer und 250 bis 1000 mg eines nicht-peptidischen Endothelin-Antagonisten enthält.

5. Präparat nach einem der Ansprüche 1 - 4, dadurch gekennzeichnet, dass das Gesamtgewicht von RAS-Hemmer aus der Gruppe von ACE-Hemmer und nicht-peptidischen Endothelin-Antagonist maximal 500 mg beträgt.

6. Handelspackung, enthaltend Cilazapril und 4-tert-Butyl-N-[6-(2-hydroxyethoxy)-5-(2-methoxyphenoxy)-2,2'-bipyrimidin-4-yl]benzolsulfonamid zusammen mit Instruktionen für die Verwendung dieser Wirkstoffe in Kombination zur gleichzeitigen, getrennten oder zeitlich abgestuften Anwendung in der Bekämpfung bzw. Verhütung von Herz-Kreislauf-Erkrankungen, insbesondere von Hypertension und deren Folgeerkrankungen sowie von Herzinsuffizienz.

## Claims

1. A preparation, characterized in that it contains 4-tert-butyl-N-[(6-(2-hydroxyethoxy)-5-(2-methoxyphenoxy)-2,2'-bipyrimidin-4-yl]benzenesulphonamide and cilazapril.

2. A preparation in accordance with claim 1 for use in the control or prevention of hypertension and disorders resulting therefrom as well as in the treatment of cardiac insufficiency.

3. A preparation according to either of claims 1-2, characterized in that the weight ratio of RAS inhibitor from the ACE inhibitor group to the non-peptidic endothelin antagonist is 1:1 to 1:500.

4. A preparation according to any one of claims 1-3, characterized in that it contains 2.5 to 10 mg of a RAS inhibitor from the ACE inhibitor group and 250 to 1000 mg of a non-peptidic endothelin antagonist.

5. A preparation according to any one of claims 1-4, characterized in that the total weight of RAS inhibitor from the ACE inhibitor group and non-peptidic endothelin antagonist is a maximum of 500 mg.

6. A commercial pack containing cilazapril and 4-tert-butyl-N-[(6-(2-hydroxyethoxy)-5-(2-methoxyphenoxy)-2,2'-bipyrimidin-4-yl]benzenesulphonamide together with instructions for the use of these active substances in combination for the simultaneous, separate or chronologically spaced use in the control or prevention of cardiovascular disorders, especially of hypertension and disorders resulting therefrom as well as of cardiac insufficiency.

## Revendications

1. Préparation, caractérisée en ce qu'elle contient du 4-tert-butyl-N-[6-(2-hydroxyéthoxy)-5-(2-méthoxyphénoxy)-2,2'-bipyrimidine-4-yl]-benzènesulfonamide et du cilazapril.

2. Préparation selon la revendication 1, pour utilisation dans la maîtrise ou la prévention de l'hypertension et de ses séquelles, et dans le traitement de l'insuffisance cardiaque.

3. Préparation selon l'une des revendications 1-2, caractérisée en ce que le rapport en poids entre l'inhibiteur du système rénine-angiotensine SRA du groupe des inhibiteurs de l'enzyme de conversion de l'angiotensine ECA, et les antagonistes non-peptidiques de l'endothéline, est de 1:1 à 1:500.

4. Préparation selon l'une des revendications 1-3, caractérisée en ce qu'elle contient de 2,5 à 10 mg d'un inhibiteur du SRA du groupe des inhibiteurs de l'ECA, et de 250 à 1000 mg d'un antagoniste non-peptidique de l'endothéline.

5. Préparation selon l'une des revendications 1 à 4, caractérisée en ce que le poids total de l'inhibiteur du SRA du groupe des inhibiteurs de l'ECA, et de l'antagoniste non-peptidique de l'endothéline, est au maximum de 500 mg.

6. Conditionnement du commerce contenant du cilazapril et du 4-tert-butyl-N-[6-(2-hydroxyéthoxy)-5-(2-méthoxyphénoxy)-2,2'-bipyrimidine-4-yl]benzènesulfonamide, en même temps que des instructions relatives à l'utilisation de ces principes actifs en combinaison avec une utilisation simultanée, distincte ou étalée dans le temps, dans la maîtrise ou la prévention de maladies cardio-vasculaires, en particulier de l'hypertension et de ses séquelles, ainsi que de l'insuffisance cardiaque.
